# EUROPEAN PATENT APPLICATION

(11) **EP 4 691 535 A1**
(43) Date of publication of application: **11.02.2026**
(21) Application number: 24779327.6
(22) Date of filing: 11.03.2024
(51) Int. Cl.: A61M 25/00, A61M 25/088

(54) **CATHETER SET**

(30) Priority: 29.03.2023 JP 2023053413; 27.02.2024 JP 2024027221
(71) Applicant: SB-Kawasumi Laboratories, Inc., Kawasaki-shi, Kanagawa 210-8602 (JP); Sumitomo Bakelite Co., Ltd., Shinagawa-ku Tokyo 140-0002 (JP)
(72) Inventor: FUJITA Yasuhiro, Kawasaki-shi, Kanagawa 210-8602 (JP)
(74) Representative: Müller-Boré & Partner Patentanwälte PartG mbB
(86) International application number: PCT/JP2024/009246
(87) International publication number: WO 2024/203196

(57) **Abstract**

The catheter set 1 includes a catheter 100, a hub 200, and an extension member 300. The catheter 100 includes a main body portion 110 and a connection portion 120. The main body portion 110 is long, and a lumen 111 is internally formed as a through-hole. A connection portion 120 is formed in a proximal end of the main body portion. The hub 200 includes a hollow portion 210 that communicates with the lumen 111 when the hub 200 is attached to the main body portion 110. The extension member 300 is long. The hub 200 is detachable from the main body portion 110 and is attachable to the main body portion 110 via the connection portion 120. Furthermore, the extension member 300 is detachable from the main body portion 110 and is attachable to the main body portion 110 via the connection portion 120.

## Description

### Technical Field

The present invention relates to a catheter set.

### Background Art

In a case of a catheter inserted into a body cavity, a separate member may be disposed to cover an outer layer side of the catheter in some cases.

For example, when a distal end of the catheter is allowed to enter a part in the body cavity, two or more catheters may be used in some cases. With regard to this type of technology, Patent Document 1 discloses a triaxial system using three catheters of a catheter (506), an intermediate catheter (582), and a controlled catheter (502). Specifically, the catheter (506) has a hollow lumen that accommodates the intermediate catheter (582), and the intermediate catheter (582) has a hollow lumen that accommodates the controlled catheter (502). In this catheter system, the intermediate catheter is disposed to cover an outer layer side of the controlled catheter (502), and the catheter (506) is disposed to cover an outer layer side of the intermediate catheter (582). In this way, three catheters are disposed to overlap each other, and each of the catheters is stretched in order, a catheter distal end reaches a desired position in the body cavity.

Alternatively, another member such as a balloon catheter may be disposed to cover an outer layer side of the catheter entering the body cavity in some cases.

### Prior Art Document

### Patent Document

[Patent Document 1] JP-A-2022-166071

### Summary of Invention

### Technical Problem

When the catheter is inserted into the body cavity, a hub for connecting a syringe for injecting a drug may be provided in a proximal end of the catheter. A general hub has a shape larger than a cross section of the catheter. However, the cross section of a separate member (for example, another catheter or the balloon catheter) delivered to the outer layer side of the catheter needs to have a small shape such that the separate member is inserted into the body cavity. Therefore, when the separate member is to be delivered to the outer layer side of the catheter, a large-diameter hub cannot pass through a through-hole provided in the separate member. Therefore, when the separate member is caused to cover the outer layer side of the catheter, it is necessary to remove the catheter once from the body cavity and reinsert the catheter, or it is necessary to use a separate member having a special mechanism.

The present invention has been made in view of the above-described problems, and an object of the present invention is to provide a catheter set that can easily deliver a separate member to an outer layer side of a catheter.

### Solution to Problem

According to the present invention, there is provided a catheter set including a catheter including a long main body portion in which a lumen is internally formed as a through-hole, and a connection portion formed in a proximal end of the main body portion, a hub detachable from the main body portion and attachable to the main body portion via the connection portion and having a hollow portion communicating with the lumen when the hub is attached to the main body portion, and a long extension member detachable from the main body portion and attachable to the main body portion via the connection portion.

The hub or the extension member is attached to the same portion of the catheter or is detached from the same portion. In this manner, when a separate member is delivered to the outer layer side of the catheter, the hub is detached from the main body portion, and the extension member is attached to the main body portion. In this way, the separate member can be disposed to be delivered to the outer layer side of the catheter without interfering with the hub, and can be moved to a distal end side of the catheter.

### Advantageous Effects of Invention

According to a catheter set of the present invention, without performing a step of removing the catheter or the like or without using a separate member having a special mechanism, the separate member can be delivered to an outer layer side of a catheter in a state where the catheter is placed in a body cavity. In this manner, the separate member can be easily delivered to the outer layer side of the catheter.

### Brief Description of Drawings

FIG. 1 is a schematic perspective view showing an example of a catheter set according to a first embodiment of the present invention.
FIG. 2A is a side view when a hub is attached to a catheter according to the first embodiment. A lumen, a hollow portion, a connection portion, and a hub-side female screw portion are indicated by a dotted line. FIG. 2B is an enlarged view of a range indicated by a two-dot chain line in FIG. 2A.
FIG. 3 is a side view when an extension member is attached to the catheter according to the first embodiment. The lumen, the connection portion, and an extension-side female screw portion are indicated by a dotted line.
FIG. 4 is a view for describing a case where the catheter set according to the first embodiment is used. Description of Embodiments

Various components such as a catheter, a hub, and an extension member which form a catheter set of the present invention need not to exist independently, and the present invention allows that a plurality of components are formed as one member, one component is formed of a plurality of members, one component is a part of another component, and a part of one component and a part of another component overlap each other.

In addition, in some cases, a method of using the catheter set according to the present invention may be described by using a plurality of steps described in order. However, the order of the description does not limit the order or a timing of performing the plurality of steps. Therefore, when the method of using the catheter set according to the present invention is performed, the order of the plurality of steps can be changed as long as there is no hindrance in terms of content, and some or all of the timings for performing the plurality of steps may overlap with each other.

Hereinafter, embodiments of the present invention will be described with reference to the drawings. In addition, in each drawing, common reference numerals will be assigned to corresponding components, and repeated description will be omitted as appropriate.

In addition, a plane referred to herein in the present invention means a shape that is physically formed by targeting a plane, and as a matter of course, it is not necessary that the plane is a geometrically perfect plane.

### <First Embodiment>

### (Catheter Set)

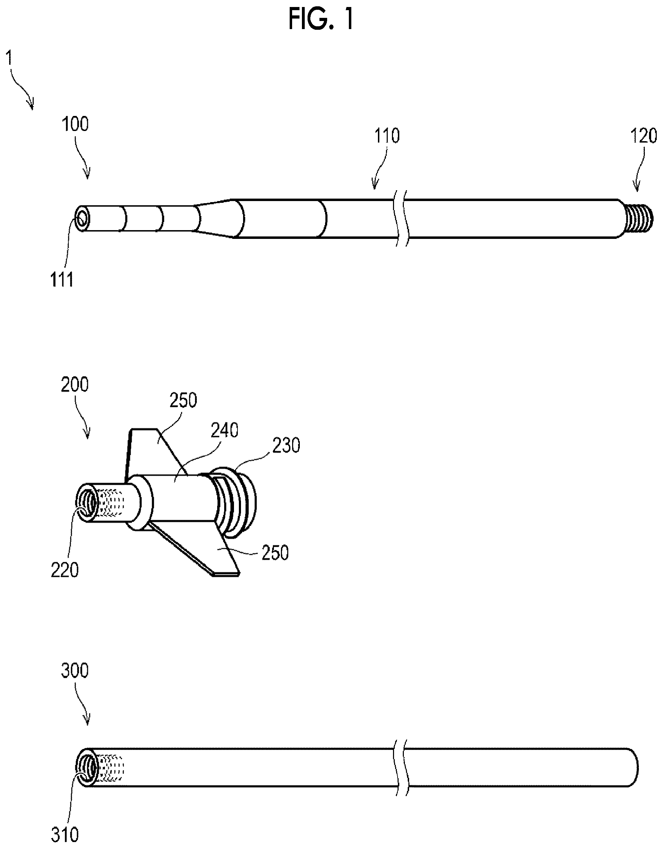

FIG. 1 is a schematic perspective view showing an example of a catheter set 1 according to a first embodiment of the present invention.

First, an outline of the catheter set 1 of the present embodiment will be described.

The catheter set 1 includes a catheter 100, a hub 200, and an extension member 300. The catheter 100 includes a main body portion 110 and a connection portion 120.

The main body portion 110 is long, and a lumen 111 is internally formed as a through-hole. The hub 200 includes a hollow portion 210 that communicates with the lumen 111 when the hub 200 is attached to the main body portion 110. The extension member 300 is long.

Next, the catheter set 1 according to the present embodiment will be described in detail.

The catheter set 1 includes the catheter 100 and components which can be used when the catheter 100 is used. The catheter 100 and the components (hub 200, extension member 300, and the like in the present embodiment) which can be used when the catheter 100 is used may be provided in a state where at least some of the components and the catheter 100 are attached, or may be separately provided in a state where the components and the catheter 100 are detached.

The catheter 100 is a long medical device that is inserted into a body cavity. For example, the catheter 100 is inserted into the body cavity such as a blood vessel, and reaches a desired position. The catheter 100 is used to adjust a flow rate inside the body cavity by injecting a drug at the desired position, expanding the blood vessel at the desired position, or the like. Hereinafter, one end inserted into the body cavity in both ends of the catheter 100 and the main body portion 110 (to be described later) will be referred to as a distal end, and the one end side will be referred to as a distal end side. In addition, the other end on a side opposite to the distal end will be referred to as a proximal end, and the other end side will be referred to as a proximal end side.

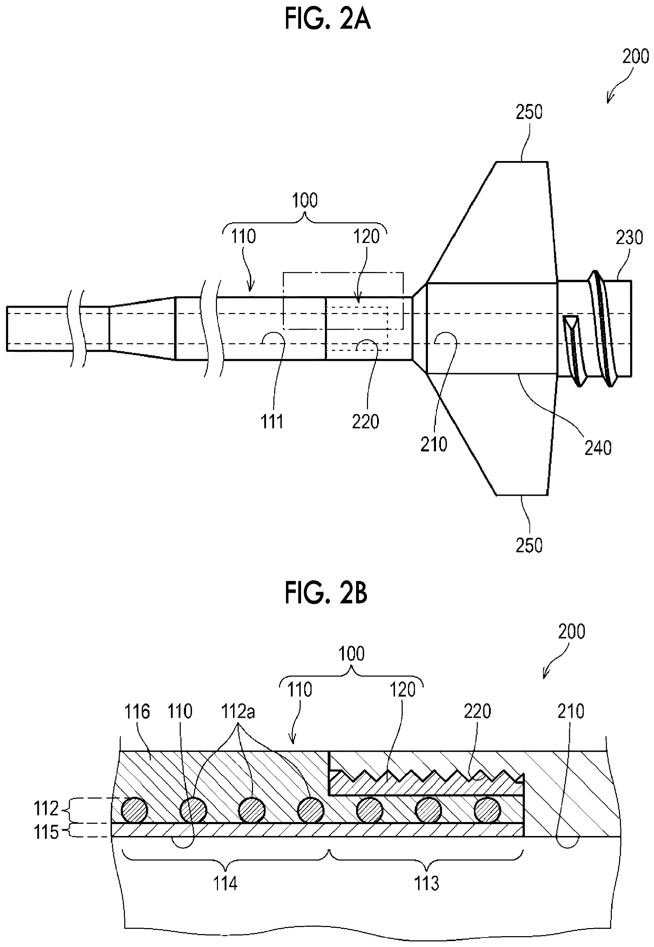

The main body portion 110 is a portion of the catheter 100 inserted into the body cavity, and is a flexible portion. The entire main body portion 110 does not need to be inserted into the body cavity, and at least a portion on the distal end side in the main body portion 110 is inserted into the body cavity. As shown in FIG. 2A, the main body portion 110 in the present embodiment has a shape and a dimension in which a cross section of a partial length region on the distal end side is larger than a cross section of a partial length region on the proximal end side. Here, in a case of a cross section of a long member, the cross section means a cross section in a plane perpendicular to a longitudinal direction of the member. Hereinafter, the longitudinal direction of the main body portion 110 and the catheter 100 will be referred to as an axial direction of the main body portion 110 or the catheter 100, or simply an axial direction.

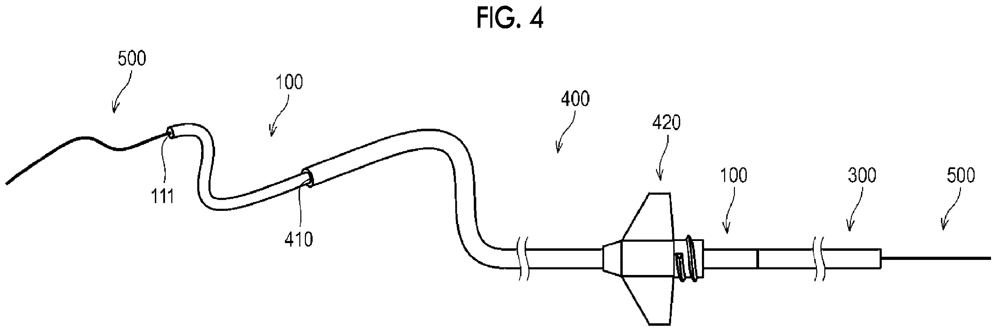

As described above, the main body portion 110 includes the lumen 111 internally formed as a through-hole. The lumen 111 extends in the axial direction, and is open in the proximal end and the distal end. A liquid agent such as a drug can be injected from the hub 200 (to be described later) to the distal end of the main body portion 110 through the lumen 111. Alternatively, as shown in FIG. 4, a guide wire 500 or a catheter having a small diameter can be inserted into the lumen 111.

The hub 200 shown in FIG. 1 is a component for connecting a syringe (not shown) for injecting a liquid agent such as a drug containing a contrast agent. In general, the syringe is filled with the liquid agent. The hub 200 includes a syringe mounting portion 230, and is connected to a syringe in the syringe mounting portion 230. In the present embodiment, the syringe mounting portion 230 is provided in the proximal end of a cylindrical portion 240 (to be described later). The hub 200 (particularly, the syringe mounting portion 230) and the syringe may be directly connected to each other, or may be indirectly connected to each other. For example, the hub 200 and the syringe may be indirectly connected to each other via a short or long tube.

The hub 200 has a cross section having a shape and a dimension which are larger than those of a cross section of the catheter 100. The cross section of the hub 200 is a cross section of the hub 200 in a plane perpendicular to the axial direction of the catheter 100 when the hub 200 is connected to the catheter 100. In addition, the hub 200 has a cross section having a shape and a dimension which are larger than those of a cross section of a second lumen 410 (refer to FIG. 4) provided in a second catheter 400 (to be described later). In the present embodiment, the hub 200 includes a cylindrical portion 240 in which a hollow portion 210 (to be described later) is formed. A radius of the cross section of the cylindrical portion 240 is larger than a radius of the cross section of the catheter 100. In addition, it is preferable that the radius of the cross section of the cylindrical portion 240 is larger than the radius of the cross section of the second lumen 410. Furthermore, the hub 200 of the present embodiment includes a pair of plate-shaped wing portions 250. The wing portion 250 is provided on a surface of the cylindrical portion 240, and protrudes in the radial direction of the catheter 100. A maximum dimension of the hub 200 in a protruding direction of the wing portion 250 is larger than a diameter in the cross section of the second lumen 410 of the second catheter 400 (to be described later). The radial direction of the catheter 100 (main body portion 110) is a direction from an axis of the catheter 100 (main body portion 110) toward a peripheral surface of the catheter 100 (main body portion 110). Regardless of whether the shape of the cross section of the catheter 100 or the main body portion 110 is a circular shape or a polygonal shape, the direction will be referred to as the radial direction of the catheter 100 or the main body portion 110.

The hollow portion 210 is a cavity portion for allowing the lumen 111 in the catheter 100 and an internal space (not shown) which stores the liquid agent inside the syringe to communicate with each other. Therefore, the hollow portion 210 of the present embodiment is open in the hub-side female screw portion 220 for connection to the catheter 100, and is open in the syringe mounting portion 230 for connection to the syringe. That is, the hollow portion 210 of the present embodiment is an internal space in which the hollow portion 210 is formed as a through-hole from the distal end to the proximal end.

The extension member 300 is a long member. The extension member 300 in the present embodiment is mainly used to be delivered to the outer layer side of the catheter 100 as will be described later. The shape of the cross section of the extension member 300 in the present embodiment is circular, but may be another shape such as a polygonal shape. Here, the outer layer side of the catheter 100 is an outer periphery of the catheter 100. More specifically, the outer layer side of the catheter 100 is the outside of the catheter 100 in the radial direction of the catheter 100.

The extension member 300 may be inserted into or may not be inserted into the body cavity. The extension member 300 may be a solid member, or may be a hollow member. For example, the extension member 300 may be an extension catheter in which a lumen is formed to extend in the longitudinal direction of the extension member 300.

As shown in FIG. 4, the catheter set 1 may include a second catheter (second catheter 400). The second catheter 400 is a long member, and is a catheter inserted into the body cavity in the same manner as the catheter 100. The second catheter 400 has a lumen (second lumen 410) formed as a through-hole. The second lumen 410 extends in the longitudinal direction of the second catheter 400. In addition, the shape of the cross section of the second lumen 410 (cross section perpendicular to an extending direction of the second lumen 410) in the present embodiment is a circular shape.

The second lumen 410 has an inner diameter larger than an outer diameter of the extension member 300 in the cross section. In addition, the second lumen 410 has an inner diameter larger than an outer diameter of the catheter 100. In this manner, the second lumen 410 can be delivered to the outer layer side of the catheter 100 to which the extension member 300 is connected as will be described later.

A second hub 420 is provided as a hub in the proximal end of the second catheter 400 in the present embodiment. The second hub 420 may be detachable from the main body portion which is a tube portion in the second catheter 400.

As shown in FIG. 4, the catheter set 1 may be used together with the guide wire 500. The guide wire 500 is a member inserted into the body cavity in a living body, and is a long member as a whole. In particular, in order to allow the distal end of the catheter (catheter 100 or second catheter 400) to reach a predetermined portion in the body, the guide wire 500 is inserted into the blood vessel or the like before the catheter reaches the predetermined portion in the body. The cross section of the guide wire 500 has a radius smaller than the cross section of the lumen 111 of the catheter 100.

As shown in FIG. 1, the catheter 100 includes the connection portion 120 in the proximal end portion. The connection portion 120 is a portion of the catheter 100 for connecting the hub 200 or the extension member 300.

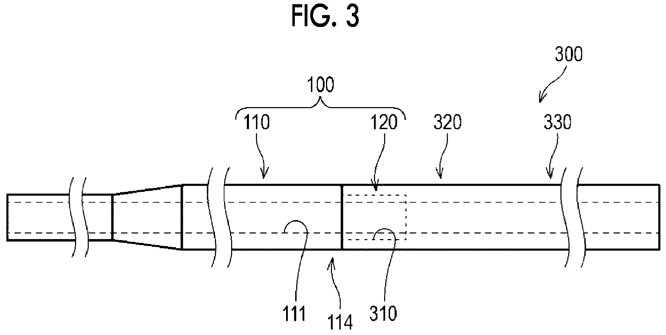

As shown in FIG. 2A, the hub 200 is detachable from the main body portion 110, and is attachable to the main body portion 110 via the connection portion 120. Furthermore, as shown in FIG. 3, the extension member 300 is detachable from the main body portion 110, and is attachable to the main body portion 110 via the connection portion 120. The description of being detachable from the main body portion 110 and attachable to the main body portion 110 means that the main body portion 110 and the component (hub 200 or extension member 300) can be attached and detached reversibly. Specifically, the description that a predetermined component is detachable from the main body portion 110 and is attachable to the main body portion 110 means that the component can be detached from the main body portion 110 after the component is attached to the main body portion 110, and that the component can be attached to the main body portion 110 after the component is detached from the main body portion 110.

A portion where the hub 200 and the extension member 300 are connected to the catheter 100 is the connection portion 120 in the catheter 100, and is the same portion in the catheter 100. That is, the hub 200 and the extension member 300 are mutually replaceable members with respect to the catheter 100.

In addition, the hub 200 and the extension member 300 have a connection target portion (hub-side female screw portion 220 or extension-side female screw portion 310, which will be described later) for connection to the connection portion 120. It is preferable that the connection target portion in the hub 200 and the connection target portion in the extension member 300 have a mutually common shape and dimension. For example, it is preferable that outer diameters of the hub-side female screw portion 220 and the extension-side female screw portion 310 (to be described later) are substantially the same. In addition, it is preferable that pitches of screw grooves are substantially the same in the hub-side female screw portion 220 and the extension-side female screw portion 310.

As shown in FIG. 2A, the hub 200 is connected to the catheter 100 such that the lumen 111 of the catheter 100 and the hollow portion 210 communicate with each other. A diameter of the proximal end portion of the lumen 111 of the catheter 100 is equal to a diameter of the distal end portion of the hollow portion 210 of the hub 200. In a state where the catheter 100 and the hub 200 are connected to each other (referred to as a connection state), it is preferable that an inner wall of the catheter 100 forming the lumen 111 and an inner wall of the hub 200 forming the hollow portion 210 are smoothly connected to each other. In this manner, generation of resistance is suppressed in a boundary between the hollow portion 210 and the main body portion 110 with respect to the liquid agent supplied from the syringe to the hollow portion 210 of the hub 200 or the guide wire 500 inserted into the hub 200. As shown in FIG. 3, the extension member 300 is attached to the catheter 100 such that the axial direction of the catheter 100 and the axial direction of the extension member 300 are coaxial with each other.

As described above, the hub 200 and the extension member 300 are mutually replaceable with respect to the connection portion 120 of the catheter 100. The hub 200 or the extension member 300 is attachable to the connection portion 120, or is detachable from the connection portion 120. In this manner, when a separate member is delivered to the outer layer side of the catheter 100, the extension member 300 can be attached by detaching the hub 200 from the main body portion 110. In this way, the separate member can be delivered to the outer layer side of the catheter 100, and can be delivered to the distal end side of the catheter 100 without interfering with the hub 200.

An example of a treatment method using the catheter 100 according to the present embodiment will be described below.

An outline of the treatment method is as follows. The treatment method using the catheter 100 is performed in a state where the catheter is placed inside the body cavity (blood vessel or the like). In the treatment method, the first catheter (catheter 100), the hub 200, the extension member 300, and an outer layer-side member (second catheter 400 or balloon catheter (not shown)) formed to be hollow and which can be delivered to the outer layer side of the catheter 100 are used.

This treatment method includes a replacement step and a moving step.

The replacement step starts in a state where the hub 200 is attached to the placed catheter 100. In the replacement step, the hub 200 is detached from the main body portion 110, and the long extension member 300 is attached to the main body portion 110. That is, the hub 200 is replaced with the extension member 300. In the moving step, the outer layer-side member is moved from the outer layer side of the catheter 100 to the outer layer side of the extension member 300, or the outer layer-side member is moved from the outer layer side of the extension member 300 to the outer layer side of the catheter 100.

Details of the treatment method will be described.

As an example of the treatment method, there may be a case where the moving step is a pulling step of pulling out the outer layer-side member from the catheter 100. More specifically, when the catheter 100 and the second catheter 400 are inserted for treatment, there may be a case where only the second catheter 400 needs to be removed from the body cavity.

Specifically, as shown in FIG. 4, the catheter set 1 (catheter set 1 and second catheter 400) is used when the distal end of the catheter 100 is allowed to enter a predetermined portion inside the body by using a so-called double coaxial system. The catheter set 1 may be used in a triple coaxial system.

In the present treatment method, the catheter 100 and the second catheter 400 to which the hub 200 is attached are inserted into the body cavity before the replacement step. In this case, preferably, the second catheter 400 is inserted first, and thereafter, the catheter 100 having the hub 200 is inserted. Specifically, the large-diameter second catheter 400 is first inserted into the body cavity through an intro-body introduction portion such as an incision, and is placed at a predetermined depth position. Thereafter, the catheter 100 is also inserted into the body cavity by inserting the small-diameter catheter 100 into an opening on the proximal end side of the second lumen 410 of the second catheter 400. The hub 200 may be inserted into the body cavity before the catheter 100 is inserted, by attaching and detaching the hub 200 to and from the catheter 100. When the second catheter 400 needs to be removed after the catheter 100 and the second catheter 400 are inserted into a proper location in the body cavity, the pulling step (moving step) is performed. In the pulling step, the second catheter 400 moves from the outer layer side of the catheter to the outer layer side of the extension member. That is, the second catheter 400 is delivered to the outer layer side of the catheter 100 and the outer layer side of the extension member 300, is removed from the body cavity, and is further pulled out from the extension member 300.

In general, when the distal end of a catheter (catheter 100 or second catheter 400) needs to reach a desired branch destination at a location (blood vessel branch location) where the blood vessel branches, the second catheter 400 may need to be removed in some cases. For example, there may be a case where the distal end of the second catheter 400 needs to be shaped in accordance with a bending shape of the blood vessel of the desired branch destination. Here, when the second catheter 400 needs to be removed from the blood vessel, the hub 200 and the second catheter 400 interfere with each other. In this case, as described above, the hub 200 is detached from the catheter 100, and is replaced with the extension member 300. In this manner, the second catheter 400 can be easily removed from the catheter 100. After the second catheter 400 is removed, the second catheter 400 whose distal end is bent at a desired angle can be inserted again into the blood vessel to cover the outer layer side of the catheter 100.

Another example of the treatment method using the catheter 100 in the present embodiment includes an example in which the above-described moving step is an outer layer-side member insertion step.

In the replacement step, after the hub 200 attached to the catheter 100 is replaced with the extension member 300, the outer layer-side member insertion step is performed. In the outer layer-side member insertion step, the outer layer-side member such as the second catheter 400 or the balloon catheter moves the outer layer side of the extension member 300 and the outer layer side of the catheter 100, and is inserted into the body cavity.

For example, when the catheter 100 to which the hub 200 is connected is inserted into the blood vessel, it may be necessary to insert the second catheter 400 into the outer layer side of the catheter 100. In this case, in order to avoid interference between the hub 200 and the second catheter 400, the hub 200 can be detached from the catheter 100, and the extension member 300 can be attached to the catheter 100.

Alternatively, it may be necessary to place the balloon catheter (not shown) when the catheter 100 is inserted into the blood vessel. Specifically, the case includes a case where the inner diameter of the blood vessel in the distal end of the catheter 100 needs to be adjusted, a case where a blood flow needs to be adjusted, a case where the distal end of the catheter 100 needs to be fixed to a desired position of the blood vessel, or the like. The balloon catheter is inserted into the blood vessel through the outer layer side of the catheter 100. When inserting the balloon catheter, the hub 200 connected to the catheter 100 is detached. In this manner, the balloon catheter does not interfere with the hub 200, and the balloon catheter can be inserted into the blood vessel.

In addition to the above-described examples, when a separate member needs to be inserted into the outer layer side of the catheter 100 or when a separate member disposed on the outer layer side of the catheter 100 needs to be removed, the insertion or the removal can be easily performed by using the catheter set 1 according to the present embodiment. That is, according to the catheter set 1 of the present embodiment, without performing a step or the like of removing the catheter 100 from the body cavity, the separate member can be easily inserted into the outer layer side of the catheter 100 or the separate member can be removed from the outer layer side of the catheter 100 in a state where the catheter 100 is placed in the body cavity. In this manner, in a simple step, the separate member can be inserted into the outer layer side of the catheter 100, or the separate member can be removed from the outer layer side of the catheter 100.

The length (length dimension) of the extension member 300 in the longitudinal direction of the extension member 300 is equal to or larger than the length (length dimension) of the catheter 100 in the longitudinal direction (axial direction) of the catheter 100. Here, the description that the length dimension of the extension member 300 is equal to the length dimension of the catheter 100 means that the length dimension of the extension member 300 is equal to or larger than half of the length dimension of the catheter 100, and is equal to or smaller than twice the length dimension of the catheter 100. Preferably, the length dimension of the extension member 300 is larger than the length dimension of the catheter 100. Still more preferably, the length dimension of the extension member 300 is larger than the length of the second catheter 400 in the longitudinal direction of the second catheter 400.

In this manner, in a state where the catheter 100 and the extension member 300 are connected to each other, even in a state where the substantially entire length region of the catheter 100 is placed in the body cavity, the extension member 300 having a sufficient length is disposed outside the body. In this manner, when the second catheter 400 is inserted or removed, the second catheter 400 can be stably inserted while the proximal end or the distal end of the extension member 300 is supported.

Instead of the present embodiment, the extension member 300 may be short. Specifically, the length dimension of the extension member 300 may be smaller than half of the length dimension of the catheter 100.

As shown in FIG. 2B, the connection portion 120 is a screw groove forming portion 120. The screw groove forming portion 120 is formed on an outer periphery (outer layer side) of the proximal end portion 113 of the main body portion 110. That is, the proximal end portion 113 and the screw groove forming portion 120 are disposed to overlap each other in the radial direction of the catheter 100. The proximal end portion 113 is a partial length region including the proximal end of the catheter 100. In particular, in the present embodiment, a partial length region of the catheter 100 disposed on the inner layer side of the connection portion 120 is the proximal end portion 113.

The hub 200 and the extension member 300 include a female screw portion (hub-side female screw portion 220 or extension-side female screw portion 310) corresponding to the screw groove forming portion 120 as a connection target portion. The hub 200 includes the hub-side female screw portion 220 as a female screw portion, and the extension member 300 includes the extension-side female screw portion 310 as a female screw portion. The description that the female screw portion corresponds to the screw groove forming portion 120 means that the screw groove forming portion 120 can be screwed to the female screw portion. More specifically, a screw groove is also formed in the female screw portion having a pitch substantially the same as a pitch of the screw groove formed in the screw groove forming portion 120. In addition, the outer diameter of the screw groove forming portion 120 and the inner diameter of the female screw portion are substantially the same.

The main body portion 110 includes an inner layer 115. The inner layer 115 is a thin film formed to be hollow and forming the lumen 111. The inner layer 115 extends in the axial direction. The inner layer 115 according to the present embodiment is formed of a resin such as a nylon-based elastomer resin or a fluororesin such as polytetrafluoroethylene (PTFE).

The main body portion 110 includes a reinforcing layer 112. The reinforcing layer 112 is formed by winding a wire 112a in the longitudinal direction of the main body portion 110 as a winding axis direction. The wire 112a is wound around a periphery of the inner layer 115. The wire 112a is spirally wound. One or a plurality of the wires 112a may be spirally wound toward one direction, or a plurality of the wires 112a may be spirally wound to form a mesh. In the present embodiment, the wire 112a includes one wire strand, but the wire 112a may be a multi-thread coil including a plurality of the wire strands. In addition, in the present embodiment, the reinforcing layer 112 is formed by winding the wire 112a in one direction. Instead of the present embodiment, the reinforcing layer 112 may be configured by winding the plurality of wires 112a in different directions. For example, a mesh structure may be formed in such a manner that the plurality of wires 112a are woven with each other. In addition, the reinforcing layer 112 is not limited to a case where the reinforcing layer 112 is formed of the wire strand, and may be formed of a metal composite body to have flexibility, for example. Specifically, a hypotube formed from a metal pipe to which flexibility is added by laser processing, a metal composite body having flexibility as a whole in such a manner that a plurality of metal elements are combined to be movable with respect to each other, or the like may be used as the reinforcing layer 112.

In addition, the main body portion 110 includes an outer layer 116. The outer layer 116 is mainly formed of a nylon-based elastomer resin. The outer layer 116 is disposed to enter a gap between the respective wound wires 112a. That is, the outer layer 116 is disposed in a gap pinched between the wires 112a when viewed in the axial direction. Furthermore, the outer layer 116 is disposed to cover the outer layer side of the reinforcing layer 112.

The reinforcing layer 112 is disposed from a region (adjacent portion 114) on the distal end side with respect to the proximal end portion 113 to the proximal end portion 113. The adjacent portion 114 is a partial length region of the catheter 100, and is a partial length region which is a region on the distal end side with respect to the proximal end portion 113 and which is adjacent to the proximal end portion 113. The reinforcing layer 112 is formed in at least the adjacent portion 114 and the proximal end portion 113, and the wire 112a is continuously wound from the adjacent portion 114 to the proximal end portion 113. In addition, it is preferable that the reinforcing layer 112 is formed in the partial length region on the distal end side with respect to the adjacent portion 114. More specifically, it is preferable that the reinforcing layer 112 is formed from the distal end to the proximal end portion 113 in the catheter 100. That is, it is preferable that the reinforcing layer 112 is formed in substantially the entire length region of the catheter 100.

As shown in FIG. 2B, the reinforcing layer 112 is disposed on the inner layer side of the screw groove forming portion 120.

Since the reinforcing layer 112 is provided from the adjacent portion 114 to the proximal end portion 113, the proximal end portion 113 and the adjacent portion 114 on which the screw groove forming portion 120 is formed on the outer layer side can be maintained in a substantially linear shape. In other words, the screw groove forming portion 120 can be supported by the reinforcing layer 112.

Preferably, the reinforcing layer 112 (wire 112a) disposed in the proximal end portion 113 is in contact with the connection portion 120. In other words, the reinforcing layer 112 (wire 112a) circumscribes an inner surface of the screw groove forming portion 120. At least a portion of the wire 112a disposed in the proximal end portion 113 may circumscribe the screw groove forming portion 120. Preferably, substantially the entire wire 112a disposed at the proximal end portion 113 circumscribe the screw groove forming portion 120.

Instead of the present embodiment, when the wire 112a is a multi-strand wire in which a plurality of wire strands are bundled, the wire strand disposed on the outer layer side in the plurality of wire strands may be in contact with the connection portion 120. In addition, when the reinforcing layer 112 is formed of a mesh structure or the like, a partial length region of the wire 112a disposed on the outer layer side at an intersection of at least the plurality of wires 112a may be in contact with the connection portion 120.

In addition, the outer layer 116 disposed in a gap pinched between the wires 112a in the axial direction is in contact with the connection portion 120.

Since the reinforcing layer 112 (wire 112a) is brought into contact with the screw groove forming portion 120, the reinforcing layer 112 can more satisfactorily support the screw groove forming portion 120.

Instead of the present embodiment, the outer layer 116 may be disposed to be pinched between the reinforcing layer 112 (wire 112a) and the connection portion 120 in the proximal end portion 113.

A cross section of the main body portion 110 and the extension member 300 is circular. The outer diameter of the cross section of the adjacent portion 114 is equal to the outer diameter of the cross section of the extension member 300. More specifically, the outer diameter of the cross section of the proximal end side of the adjacent portion 114 is equal to the outer diameter of the cross section of one end of the extension member 300 on which the extension-side female screw portion 310 is formed. Here, the description that the outer diameters are equal means that the outer diameter of the cross section of the adjacent portion 114 is larger than half of the outer diameter of the cross section of the extension member 300, and is smaller than twice the outer diameter. In this manner, when the catheter 100 and the extension member 300 are connected to each other as shown in FIG. 3, an outer surface of the adjacent portion 114 and an outer surface of one end portion of the extension member 300 are substantially uniform. That is, substantially no step difference is provided or only a small step difference is provided between the outer surface of the adjacent portion 114 and the outer surface of the extension member 300. When the step difference is generated between the adjacent portion 114 and the outer surface of the extension member 300, it is preferable that a height of the step difference is equal to or smaller than a thickness of the extension member 300 in which the extension-side female screw portion 310 is provided (thickness from a peripheral surface defining the extension-side female screw portion 310 to an outer peripheral surface of the extension member 300).

When the catheter 100 and the extension member 300 are connected to each other, the outer surface of the catheter 100 and the outer surface of the extension member 300 are connected to each other in an approximately flat manner. In this manner, it is easy to deliver the second catheter 400 to the outer layer side of the catheter 100 and the extension member 300, or damage to the second lumen 410 in the second catheter 400 is suppressed.

It is preferable that the adjacent portion 114 is more rigid than a partial length region on the distal end side of the adjacent portion 114 or is more rigid than the distal end portion of the main body portion 110. Here, the description that the adjacent portion 114 is more rigid than the other partial length region means that torsional rigidity of the adjacent portion 114 is stronger than torsional rigidity of the other partial length region. In other words, the description that the adjacent portion 114 is more rigid than the other partial length region means that a value obtained by multiplying an average modulus of transverse elasticity in which the reinforcing layer 112 or the resin layer (outer layer 116 or inner layer 115) is added in the adjacent portion 114 and a polar second moment of area is greater than the value in the other partial length region. In this manner, it is easy to separate the catheter 100 from the extension member 300 or the hub 200 and to screw the catheter 100 to the extension member 300 or the hub 200 by holding the adjacent portion 114 with hands.

It is preferable that the proximal end portion 113 is more rigid than the distal end portion of the main body portion 110, the adjacent portion 114, or the partial length region on the proximal end side with respect to the adjacent portion 114. Here, the description that the proximal end portion 113 is rigid means that compression stiffness of the proximal end portion 113 is stronger than that of the other partial length region. In other words, the description that the proximal end portion 113 is rigid means that an average modulus of longitudinal elasticity (Young's modulus) in which the reinforcing layer 112 or the resin layer is added in the proximal end portion 113 is higher than an average modulus of longitudinal elasticity of the other partial length region. In this manner, it is easy to form the screw groove forming portion 120 on the outer layer side of the proximal end portion 113. For example, since the proximal end portion 113 is rigid, it is easy to form the screw groove forming portion 120 to be crimped on the outer layer side of the proximal end portion 113. The outer layer 116 may be formed after the screw groove forming portion 120 is formed on the outer layer side of the reinforcing layer 112, or the screw groove forming portion 120 may be formed after the outer layer 116 and the reinforcing layer 112 are formed.

Specifically, the proximal end portion 113 or the adjacent portion 114 may be formed to be rigid by forming the adjacent portion 114 or the proximal end portion 113 with the multi-strand wire 112a or by winding the plurality of wires 112a in different directions. Alternatively, the outer layer 116 forming the proximal end portion 113 or the adjacent portion 114 may be formed of a material harder than the outer layer 116 forming the distal end portion or the like.

Furthermore, it is preferable that the proximal end portion 113 and the adjacent portion 114 are integrally formed to be rigid. In other words, it is preferable that the proximal end portion 113 and the adjacent portion 114 are less likely to be bent or twisted at a boundary between the proximal end portion 113 and the adjacent portion 114. In this manner, it is easy to detach the catheter 100 and the hub 200 or the extension member 300 by holding the adjacent portion 114. For example, the rigid reinforcing layer 112 configured to include the multi-strand wire 112a or the plurality of wires 112a wound in different directions may be continuously delivered from the proximal end portion 113 to the adjacent portion 114 without a seam. Alternatively, the outer layer 116 is formed of a hard material and is integrated may be disposed in the proximal end portion 113 and the adjacent portion 114. Instead of integrally forming the proximal end portion 113 and the adjacent portion 114 to be rigid, the proximal end portion 113 formed to be rigid and the adjacent portion 114 formed to be rigid may be separately formed. In this case, the proximal end portion 113 and the adjacent portion 114 may be disposed in contact with each other, or may be disposed apart from each other while being close to each other. When the proximal end portion 113 and the adjacent portion 114 which are each formed to be rigid are disposed apart from each other in the axial direction, an upper limit of a separation distance therebetween is not limited, but it is preferably that the upper limit is smaller than the diameter of the main body portion 110.

The screw groove forming portion 120 may be configured to be rigid integrally with the adjacent portion 114. In other words, it is preferable that the screw groove forming portion 120 and the adjacent portion 114 are less likely to be bent or twisted at a boundary between the screw groove forming portion 120 and the adjacent portion 114. Here, the description that the screw groove forming portion 120 is rigid means that torsional rigidity of the screw groove forming portion 120 is sufficiently strong. That is, the entire torsional rigidity of the screw groove forming portion 120 and the adjacent portion 114 is strong. In this manner, it is easy to screw the hub 200 or the extension member 300 to the catheter 100 or to detach the hub 200 or the extension member 300 from the catheter 100. For example, the screw groove forming portion 120 may be formed of the same material as that of the hard outer layer 116 forming the adjacent portion 114, and the screw groove forming portion 120 and the adjacent portion 114 may be integrally formed. Alternatively, a portion of the screw groove forming portion 120 may be embedded in the hard outer layer 116. In this manner, the screw groove forming portion 120 and the adjacent portion 114 may be integrally rigid. Instead of integrally forming the screw groove forming portion 120 and the adjacent portion 114 to be rigid, the screw groove forming portion 120 formed to be rigid and the adjacent portion 114 formed to be rigid may be separately formed. In this case, both may be disposed in contact with each other, or may be disposed apart from each other while being close to each other. When the screw groove forming portion 120 formed to be rigid and the adjacent portion 114 formed to be rigid are close to each other, the upper limit of the separation distance therebetween is not limited, but specifically, it is preferable that the separation distance is smaller than the diameter of the main body portion 110.

In addition, it is preferable that the torsional rigidity of one end portion (referred to as a distal end portion of the extension member 300) in which an extension-side female screw portion 310 of the extension member 300 is formed is stronger than the torsional rigidity of an intermediate portion or the other end portion. Alternatively, it is preferable that the torsional rigidity of the distal end portion of the extension member 300 is stronger than the torsional rigidity of the main body portion 110 (particularly, the adjacent portion 114 or the proximal end portion 113). In this manner, it is easy to separate the extension member 300 from the catheter 100 while the extension member 300 is rotated with respect to the catheter 100.

As shown in FIG. 3, the second adjacent portion 320 is a portion of the extension member 300, and is a portion adjacent to the proximal end side of the extension-side female screw portion 310. It is preferable that the second adjacent portion 320 and the extension-side female screw portion 310 are integrally formed. In this manner, since the second adjacent portion 320 and the extension-side female screw portion 310 have sufficient torsional rigidity, it is easy to attach the extension member 300 to the catheter 100 or to detach the extension member 300 from the catheter 100.

In addition, a portion (center portion 330) on the proximal end side with respect to the second adjacent portion 320 in the extension member 300 may have weaker torsional rigidity than the second adjacent portion 320 and the extension-side female screw portion 310. Since the center portion 330 is more likely to be twisted than the second adjacent portion 320 and the extension-side female screw portion 310, the center portion 330 can be allowed to be temporarily twisted in a fixed level, when the extension member 300 is screwed to the catheter 100. In this manner, when the extension member 300 is screwed to the catheter 100, a possibility that the extension member 300 (particularly, the center portion 330) is unexpectedly and largely moved due to the twisting is suppressed, and even when the extension member 300 is unexpectedly moved, the movement becomes smaller. Therefore, it is easy to operate the extension member 300. In addition, since the center portion 330 is allowed to be temporarily twisted in a fixed level, even when an unexpected external force is applied to the extension member 300 and the catheter 100 to release fitting therebetween, the extension member 300 is less likely to be detached from the catheter 100, and operability of the catheter set 1 can be improved.

Alternatively, the second adjacent portion 320 may be coupled to the center portion 330 to be rotatable in the circumferential direction. In other words, the center portion 330 may be coupled to the second adjacent portion 320 to be rotatable in the circumferential direction. In this manner, when the second adjacent portion 320 is rotated to screw the extension-side female screw portion 310 to the catheter 100, the center portion 330 can be pressed by a jig or a hand while the second adjacent portion 320 is rotated, and the rotation of the center portion 330 in the circumferential direction can be suppressed. In this manner, it is possible to suppress a possibility that the center portion 330 is unexpectedly moved when the extension member 300 is screwed to the catheter 100. Specifically, a groove (fitting groove) extending in the circumferential direction may be formed on an outer peripheral surface of the second adjacent portion 320, a protrusion extending in the circumferential direction may be formed on an inner wall of the center portion 330 to correspond to the fitting groove, and the protrusion may engage with the fitting groove. In this manner, the second adjacent portion 320 and the center portion 330 are connected while being less likely to be separated from each other in the axial direction, but the second adjacent portion 320 is relatively rotatable with respect to the center portion 330. The present invention is not limited to the above-described aspect, and the second adjacent portion 320 may be coupled to the center portion 330 to be rotatable in the circumferential direction by using another method.

In order to suppress a possibility that the extension member 300 is unexpectedly moved when the extension member 300 is screwed to the catheter 100, the extension member 300 may be divided into a plurality of short members. The short members are connected to each other by engagement such as screwing. In this case, when the extension member 300 is screwed to the catheter 100, first, the first short member is screwed to the catheter 100, and thereafter, the second short member is screwed to the first short member. In this way, when the short members are screwed in order, the members to be screwed are short. Therefore, a possibility that the members are unexpectedly moved due to twisting during screwing is suppressed. In addition, the extension member 300 can have a desired length by joining the plurality of short members.

As shown in FIGS. 2A and 2B, the outer diameter of the proximal end portion 113 of the main body portion 110 is smaller than the outer diameter of the adjacent portion 114. That is, as shown in FIG. 2B, a step difference descending from the adjacent portion 114 to the proximal end portion 113 is provided between the proximal end portion 113 and the adjacent portion 114. It is preferable that the height of the step difference (dimension of the catheter 100 in the radial direction) is larger than the thickness of the connection portion 120 (dimension of the catheter 100 in the radial direction). The outer diameter of the cross section of the connection portion 120 is equal to or smaller than the outer diameter of the cross section of the adjacent portion 114. In other words, a screw thread of the screw groove forming portion 120 coincides with an outer edge of the adjacent portion 114 or is disposed inside the main body portion 110 with respect to the outer edge, when viewed in the axial direction. Preferably, the outer diameter of the cross section of the connection portion 120 is smaller than the outer diameter of the cross section of the adjacent portion 114. However, in the present invention, an example is not limited to a configuration in which the outer diameter of the adjacent portion 114 and the outer diameter of the connection portion 120 completely coincide with each other and the outer surfaces of the adjacent portion 114 and the connection portion 120 are formed to be flat. The present invention does not exclude a configuration in which a slight ascending step difference or a slight descending step difference is provided from the adjacent portion 114 to the connection portion 120. That is, the outer diameter of the connection portion 120 may be slightly larger than the outer diameter of the adjacent portion 114. When the connection portion 120 is the screw groove forming portion 120, the outer diameter of the cross section of the screw groove forming portion 120 is the outer diameter based on a top portion of a screw thread of the screw groove forming portion 120. The description that the outer diameter of the cross section of the connection portion 120 is equal to the outer diameter of the cross section of the adjacent portion 114 means that the outer diameter of the cross section of the connection portion 120 is equal to or larger than half of the outer diameter of the cross section of the adjacent portion 114, and is equal to or smaller than twice the outer diameter.

Since the outer diameter of the connection portion 120 is equal to or smaller than the outer diameter of the adjacent portion 114, when the hub 200 or the extension member 300 is connected to the catheter 100, it is possible to suppress a possibility that a portion of the hub 200 or the extension member 300 covering the outer layer side of the connection portion 120 protrudes in the radial direction of the catheter 100.

An outer surface of the catheter 100 (particularly, the adjacent portion 114) and an outer surface of the extension member 300 (particularly, the distal end portion) may be colored with different chromatic colors. In this manner, it is easy to visually recognize a connection portion between the extension member 300 and the catheter 100 when the extension member 300 is detached from the catheter 100.

The present invention is not limited to the above-described embodiment, and various modifications, improvements, and the like are also included as long as the object of the present invention is achieved.

The following modification examples can be appropriately combined.

In the present embodiment, as described above, the connection target portion in the hub 200 or the extension member 300 and the connection portion 120 of the catheter 100 are connected by screwing, but an aspect of the connection between the connection target portion and the connection portion 120 is not limited thereto. Instead of the present embodiment, the connection target portion and the connection portion 120 in the hub 200 or the extension member 300 may be connected by a structure that is not a screw structure.

For example, the connection target portion and the connection portion 120 may have uneven engagement portions that engage with each other, and the connection target portion and the connection portion 120 may be connected to each other by engagement. In addition, the connection target portion and the connection portion 120 may maintain the connection by pressure contact therebetween.

The embodiment includes the following technical ideas.
(1) There is provided a catheter set including a catheter including a long main body portion in which a lumen is internally formed as a through-hole, and a connection portion formed in a proximal end of the main body portion, a hub detachable from the main body portion and attachable to the main body portion via the connection portion and having a hollow portion communicating with the lumen when the hub is attached to the main body portion, and a long extension member detachable from the main body portion and attachable to the main body portion via the connection portion.
(2) In the catheter set according to (1), a length of the extension member in a longitudinal direction of the extension member is equal to or larger than a length of the catheter in a longitudinal direction of the catheter.
(3) In the catheter set according to (2), the connection portion is a screw groove forming portion formed on an outer periphery of a proximal end portion of the main body portion. The hub and the extension member have female screw portions corresponding to the screw groove forming portion. The main body portion includes a reinforcing layer around which a wire is wound by using a longitudinal direction of the main body portion as a winding axis direction. The reinforcing layer is disposed from a region on a distal end side with respect to the proximal end portion to the proximal end portion.
(4) In the catheter set according to (3), cross sections of the main body portion and the extension member are circular, and an outer diameter of a partial length region adjacent to the proximal end portion, which is the region on the distal end side in the catheter, is equal to an outer diameter of the extension member.
(5) In the catheter set according to (4), an outer diameter of the proximal end portion of the main body portion is smaller than the outer diameter of the partial length region, and an outer diameter of the connection portion is equal to or smaller than the outer diameter of the partial length region.
(6) In the catheter set according to (5), the reinforcing layer disposed in the proximal end portion is in contact with the connection portion.
(7) The catheter set according to (6) further includes a second catheter in which a lumen having an inner diameter larger than the outer diameter of the extension member is formed as a through-hole.
(8) In the catheter set according to (7), a length of the extension member is larger than the length of the second catheter.
(9) There is provided a treatment method performed in a state where a catheter is placed in a body cavity. The treatment method includes a step of using a catheter including a long main body portion in which a lumen is internally formed as a through-hole, and a connection portion formed in a proximal end of the main body portion, a hub detachable from the main body portion and attachable to the main body portion via the connection portion and having a hollow portion communicating with the lumen when the hub is attached to the main body portion, a long extension member detachable from the main body portion and attachable to the main body portion via the connection portion, and an outer layer-side member formed to be hollow and deliverable to the outer layer side of the catheter, a replacement step of detaching the hub from the main body portion and attaching the long extension member to the main body portion in a state where the hub is attached to the placed catheter, and a moving step of moving the outer layer-side member from the outer layer side of the catheter to the outer layer side of the extension member or moving the outer layer-side member from the outer layer side of the extension member to the outer layer side of the catheter.
(10) The treatment method according to (9) further includes an insertion step of inserting the catheter and the second catheter into the body cavity before the replacement step. The moving step is a pulling step of moving the outer layer-side member from the outer layer side of the catheter to the outer layer side of the extension member and pulling out the outer layer-side member from the extension member.
(11) In the treatment method according to (9), the moving step is an outer layer-side member insertion step of moving the outer layer-side member to the outer layer side of the extension member and the outer layer side of the catheter and inserting the outer layer-side member into the body cavity.

### Reference Signs List

- 1:: catheter set
- 100:: catheter
- 110:: main body portion
- 111:: lumen
- 112:: reinforcing layer
- 112a:: wire
- 113:: proximal end portion
- 114:: adjacent portion
- 115:: inner layer
- 116:: outer layer
- 120:: connection portion, screw groove forming portion
- 200:: hub
- 210:: hollow portion
- 220:: hub-side female screw portion
- 230:: syringe mounting portion
- 240:: cylindrical portion
- 250:: wing portion
- 300:: extension member
- 310:: extension-side female screw portion
- 320:: second adjacent portion
- 330:: center portion
- 400:: second catheter
- 410:: second lumen
- 420:: second hub
- 500:: guide wire

## Claims

1. A catheter set comprising:
a catheter including a long main body portion in which a lumen is internally formed as a through-hole, and a connection portion formed in a proximal end of the main body portion;
a hub detachable from the main body portion and attachable to the main body portion via the connection portion and having a hollow portion communicating with the lumen when the hub is attached to the main body portion; and
a long extension member detachable from the main body portion and attachable to the main body portion via the connection portion.

2. The catheter set according to Claim 1,
wherein a length of the extension member in a longitudinal direction of the extension member is equal to or larger than a length of the catheter in a longitudinal direction of the catheter.

3. The catheter set according to Claim 2,
wherein the connection portion is a screw groove forming portion formed on an outer periphery of a proximal end portion of the main body portion,
the hub and the extension member have female screw portions corresponding to the screw groove forming portion,
the main body portion includes a reinforcing layer around which a wire is wound by using a longitudinal direction of the main body portion as a winding axis direction, and
the reinforcing layer is disposed from a region on a distal end side with respect to the proximal end portion to the proximal end portion.

4. The catheter set according to Claim 3,
wherein cross sections of the main body portion and the extension member are circular, and
an outer diameter of a partial length region adjacent to the proximal end portion, which is the region on the distal end side in the catheter, is equal to an outer diameter of the extension member.

5. The catheter set according to Claim 4,
wherein an outer diameter of the proximal end portion of the main body portion is smaller than the outer diameter of the partial length region, and
an outer diameter of the connection portion is equal to or smaller than the outer diameter of the partial length region.

6. The catheter set according to Claim 5,
wherein the reinforcing layer disposed in the proximal end portion is in contact with the connection portion.

7. The catheter set according to Claim 6, further comprising:
a second catheter in which a lumen having an inner diameter larger than the outer diameter of the extension member is formed as a through-hole.

8. The catheter set according to Claim 7,
wherein the length of the extension member is larger than a length of the second catheter.
